# EUROPEAN PATENT SPECIFICATION

(11) **EP 4 146 129 B1**
(45) Date of publication and mention of the grant of the patent: **29.10.2025**
(21) Application number: 21733174.3
(22) Date of filing: 30.04.2021
(51) Int. Cl.: A61F 9/02, A41D 13/11, G02B 7/02, G02C 9/04, G02C 7/16, G02B 25/00, G02C 7/08

(54) **PROTECTIVE VISOR, IN PARTICULAR FOR A SAFETY DEVICE FOR THE FACE OF A USER**
SCHUTZVISIER, INSBESONDERE FÜR EINE SICHERHEITSVORRICHTUNG FÜR DAS GESICHT EINES BENUTZERS
VISIÈRE DE PROTECTION, EN PARTICULIER POUR UN DISPOSITIF DE SÉCURITÉ POUR LE VISAGE D'UN UTILISATEUR

(30) Priority: 04.05.2020 IT 202000002119 U
(43) Date of publication of application: 15.03.2023
(73) Proprietor: PASQUANTONIO, Guido, 00187 Roma (IT)
(72) Inventor: PASQUANTONIO, Guido, 00187 Roma (IT)
(74) Representative: Puggioli, Tommaso
(86) International application number: PCT/IB2021/053621
(87) International publication number: WO 2021/224738

(56) References cited:
- DE-A1- 102013 207 474
- US-A- 4 955 394
- US-A- 5 499 064

## Description

### Technical field

This invention relates to a protective visor, in particular for a safety device for the face of a user, and a safety device for the face of a user.

### Background art

More specifically, the invention relates to the technical field of personal protective equipment for the eyes of a user.

In fact, an operator is subject to various types of risks that can put his/her eyes at risk.

For this reason, various personal protective devices have been developed, designed to protect the eyes of an operator from chippings, hot or caustic or corrosive materials, radiation, which may lead to mechanical, optical and thermal lesions.

In the medical field, for example, these devices must allow the user to operate in contaminating environments, guaranteeing that they are isolated from any infectious agents.

Amongst the common personal protective devices there are goggles, masks, visors and screens.

Moreover, these devices may be equipped with different types of filters, for example for welding, ultraviolet rays, infrared rays and solar protection in such a way as to limit damage and disturbances correlated with them.

Disadvantageously, the visors of these devices, an exemple of which is disclosed in document DE102013207474, generate impediments and reduce the comfort for the user in the use of optical instruments such as, for example, microscopes or telescopes.

In fact, the operators are often forced to remove the safety device in such a way as to be able to use the above-mentioned optical instruments, determining an increase in the operating times.

Moreover, very disadvantageously, in the situations in which the operator is operating in a potentially contaminated environment, the operator will be forced to transfer to a safe environment for using the above-mentioned optical instruments.

Disadvantageously, some protective devices, due to their special shape, do not allow the use of prescription glasses to be used by persons affected by visual defects.

Although some special devices, which have a more spacious shape, are available on the market, configured to allow an operator to wear eyeglasses under the protective device, these devices are particularly bulky and reduce the mobility of the operator during performance of his/her duties.

Moreover, these devices are particularly inconvenient because, once the protective device is worn, the user has very limited access, if not none at all, to the eyeglasses and cannot therefore vary their positioning which, generally speaking, has been modified due to the procedures for fastening the device to the face of the user.

Document US4955394 relates to a face shield comprising two sockets formed to accept and hold firmly the rims surrounding the optical lenses of a pair of eyeglasses. The sockets are formed as recesses projecting away from the eyes of the user to contain the eyeglasses.

### Aim of the invention

In this context, the technical purpose which forms the basis of the invention is to provide a protective visor, in particular for a safety device for the face of a user, and a safety device for the face of a user, which overcomes the above-mentioned drawbacks of the prior art.

More specifically, the aim of the invention is to provide a protective visor, in particular for a safety device for the face of a user, and a safety device for the face of a user which guarantees high levels of comfort for the user.

A further aim of the invention is to provide a protective visor, in particular for a safety device for the face of a user, and a safety device for the face of a user, having a high level of operational flexibility.

Another aim of the invention is to provide a protective visor, in particular for a safety device for the face of a user, and a safety device for the face of a user which is able to simplify the work and increase the operating efficiency of a user.

The technical purpose indicated and the aims specified are substantially achieved by a protective visoraccording to claim 1 and a safety device according to claim 19.

The dependent claims correspond to possible embodiments of the invention.

### Brief description of the drawings

Further features and advantages of this invention are more apparent in the non-limiting description below, with reference to a preferred, non-limiting, embodiment of a protective visor, in particular for a safety device for the face of a user, and a safety device for the face of a user as illustrated in the accompanying drawings, in which:
- Figures 1 to 5 are front views of some possible embodiments of a protective visor, according to the invention;
- Figure 6 is a side view of a possible embodiment of a protective visor according to the invention;
- Figures 7 to 8 are schematic views of the protective visor according to the invention;
- Figure 9 is a perspective view of a possible embodiment of a protective visor according to the invention;
- Figures 10-14B are partial schematic side views of some possible embodiments of a protective visor according to the invention;
- Figures 15a-15b are partial schematic side views of some possible embodiments of a protective visor according to the invention.

### Detailed description of preferred embodiments of the invention

With reference to the accompanying drawings, the numeral 1 denotes in its entirety a protective visor, in particular for a safety device 100 for the face of a user 200, which will hereinafter be referred to as the visor 1.

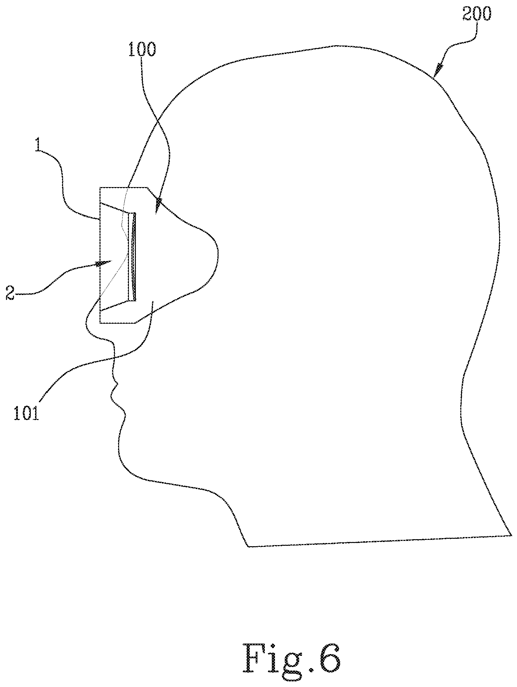

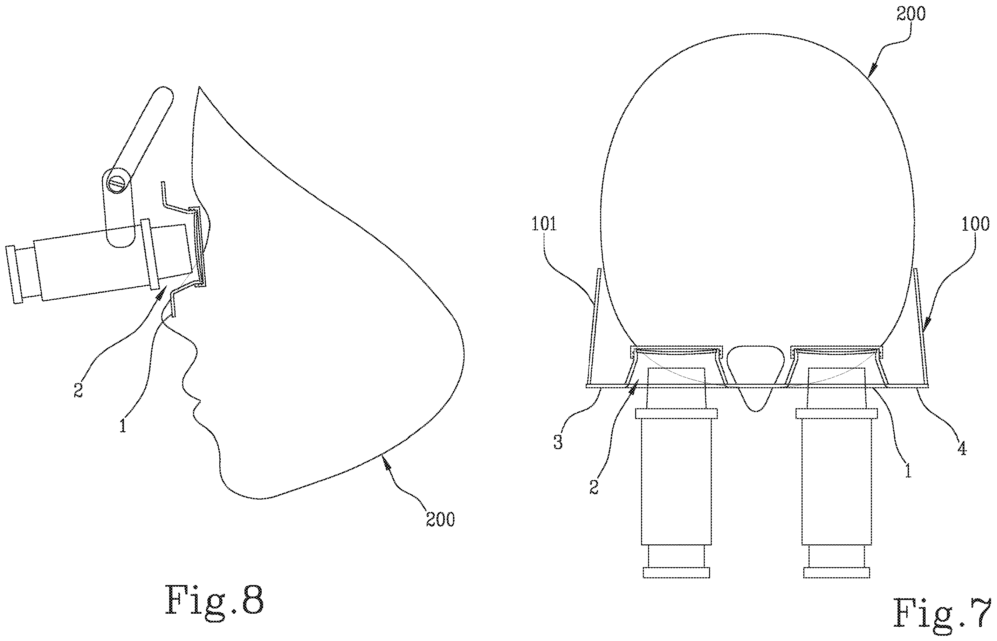

As illustrated in Figures 6, 7, the visor 1 can be connected to a supporting portion, for example a frame, of the above-mentioned safety device 100, in order to protect the eyes of the user 200 against chippings, hot or caustic or corrosive materials and/or radiation which can damage them.

The visor 1 is made of transparent material and has a pair of recesses 2 for positioning, in a position close to the eyes of the user 200, viewing and/or viewing amplification instruments (as illustrated in Figures 7, 8).

In this way, the user 200 can easily use these instruments without the need to remove the safety device 100.

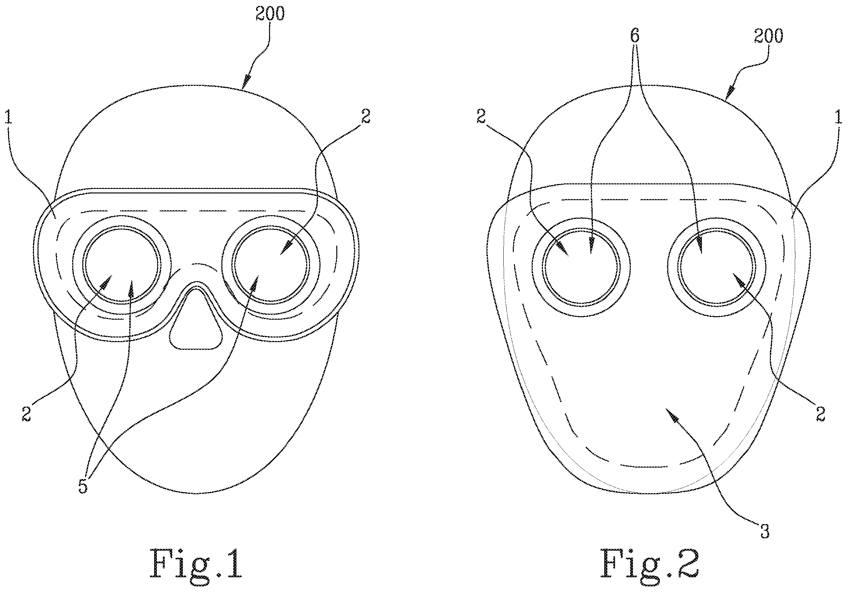

As illustrated in Figures 1 to 5, the recesses 2 may have different types of geometrical shapes, for example circular or drip, without altering the innovative concept at the base of this invention. Preferably, the visor 1 is made of polymeric material and/or by a plastic moulding process.

Advantageously, the visor 1 may have an anti-glare portion and/or with increased opacity in such a way as to improve the comfort of a user 200 during the operating steps.

The visor 1 comprises at least a first portion 3 forming at least a respective first protective surface 4 designed to protect the periocular zones of the user 200.

In one possible embodiment of the invention, the surface area of the first portion 3 is such that it can cover, in use, only the ocular and periocular zone of a user 200, like protective eyeglasses (as illustrated in Figures 1, 3-5).

More specifically, the surface extension of the first portion 3 may depend on the specific operating requirements and on the need to give the user a high level of comfort.

According to a further possible embodiment and as illustrated in Figure 2, the first portion 3 has a surface area such as to substantially cover, in use, the entire face of a user 200 without altering the innovative concept at the base of the invention.

According to that embodiment, suitable openings (not illustrated in the accompanying drawings) made on the visor 1 and/or on the safety device 100 may be provided for the face of a user 200 designed to allow an adequate breathing by the user 200.

The visor 1 comprises also a pair of second portions 5 which delimit in the visor, at least partly, a recess, towards the eyes of the user 200, relative to the first portion 3.

In one embodiment, the portions 5 form respective second protective surfaces 6 designed to protect the ocular zones of the user 200.

In one embodiment, the protective surfaces 6 are joined together, forming a single space. second portions 5 are made of substantially transparent material in such a way as to guarantee a high visibility to the user 200 during use of the visor 1.

In one embodiment, the first portion 3 may extend towards the portions 5 and/or the protective surfaces 6.

As illustrated in the accompanying drawings, the visor 1 may comprise connecting portions 7 between the first portion 3 and the second portions 5 configured to define a surface continuity of the visor forming a protection for the face of the user 200.

In other words, the second portions 5 and the connecting portions 7 form the recesses 2.

More specifically, the second portion 5 forms a recess relative to the first portion 3 having a depth of between 2 - and 50- mm, preferably 30- mm.

In use, the portion 5 protrudes from the portion 3, towards the face of the user 200, by a distance of between 2 and 50 mm, preferably by a distance of 30 mm.

Preferably, the recess 2 comprises the surfaces 6.

The surfaces 6 have, relative to the first portion 3, a depth of between 2 - and 50- mm, preferably 30- mm.

In use, the surfaces 6 protrude from the portion 3, towards the face of the user 200, by a distance of between 2 and 50 mm, preferably by a distance of 30 mm.

Preferably, the connecting portions 7 have a tubular shape or have an extension converging from the first protective surface 4 to the second protective surface 6.

In other words, the connecting portions 7 extend, preferably, transversely to the first and second portions 3, 5 and are configured to connect the first and second portions 3, 5 in such a way that during an operating configuration the second protective surface 6 is close to the user 200 relative to the first protective surface 4.

More specifically, the connecting portions 7 have a shape designed to limit, and preferably not generate, any obstruction to the field of vision of the user 200.

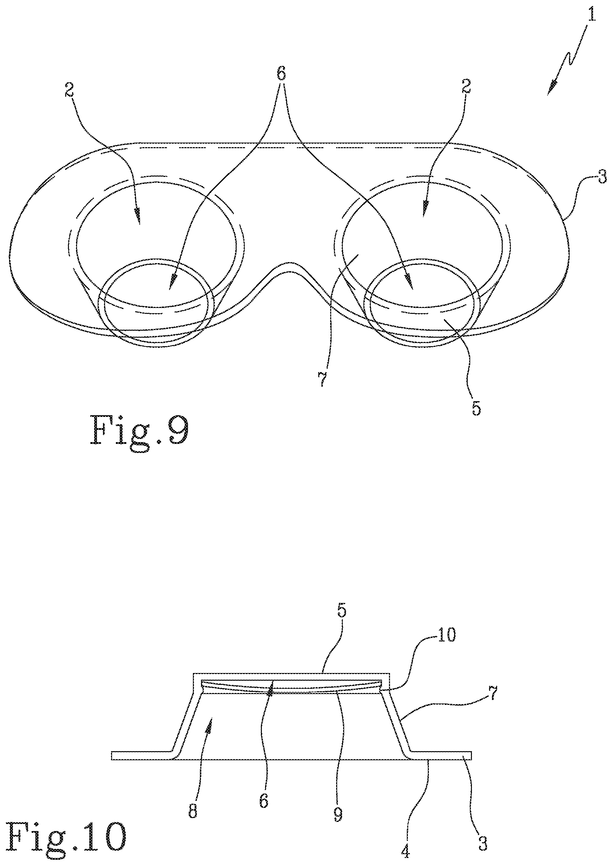

According to a possible embodiment and as illustrated in Figure 9, the connecting portions 7 are made as one piece with the second portions 5 by means of a transparent material.

In that case, the portions 5 define the protective surfaces 6.

Each connecting portion 7 may be stably applicable to the first portion 3 for example by means of a snap-on connection, by pressure, by screwing or by gluing.

In one embodiment, illustrated for example in Figure 10, the first portion 3, the connecting portions 7 and the second portions 5 are made in a single body of transparent material.

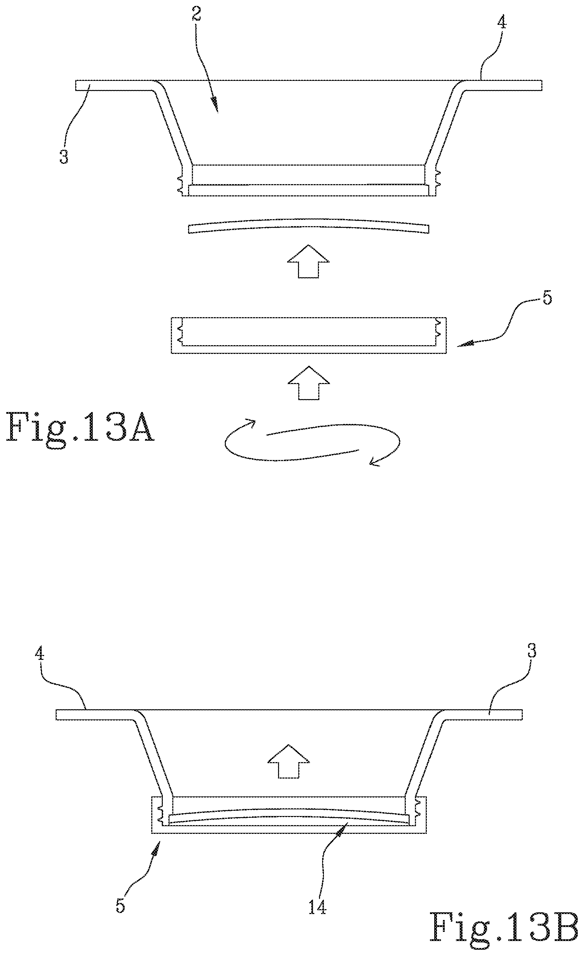

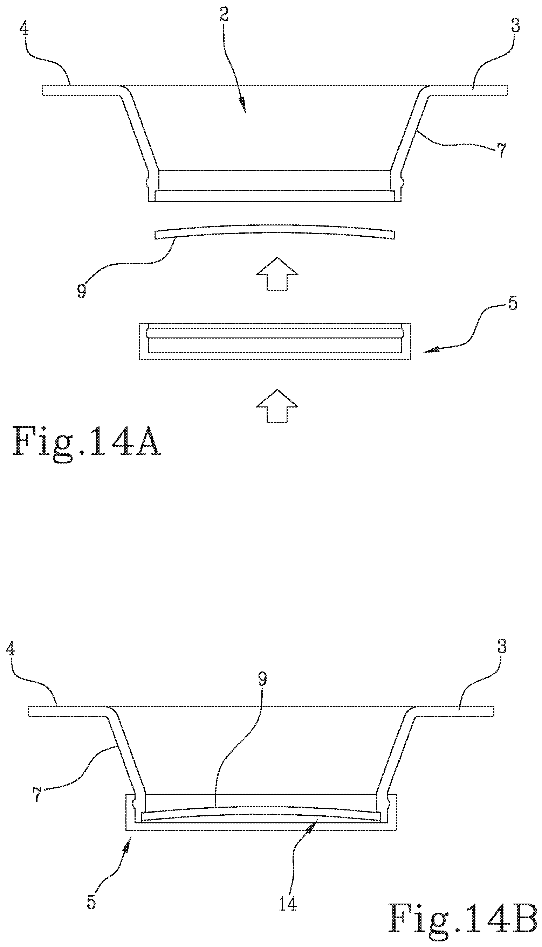

According to an embodiment, as illustrated for example in Figures 13A, 13B, 14A, 14B, the connecting portions 7 are made as one piece with the first portion 3 using a transparent material.

In one embodiment, each second portion 5 comprises or defines a respective protective surface 6 and is stably applicable to the respective connecting portion 7, for example by a snap-on connection, by pressure, by screwing or by gluing or the like.

In one embodiment, each connecting portion 7 may define a housing 8 designed to receive at least one lens 9.

The lens 9 may be, for example, a zero graduated or neutral base lens, also known as *"prescription"* or *"non-prescription lenses".*

The lens 9 may be a flat lens or a curved lens and allow the visor 1 to respond to a wide range of dioptrics.

Preferably, the housing 8 is designed to receive the above-mentioned lens 9 in a direction which extends towards the user 200 during a condition of use of the visor 1.

Preferably, the housing 8 is made at the recesses 2.

In this way, the visor 1 can support lenses, for example corrective lenses, which allow improved viewing for a user 200 affected by sight defects.

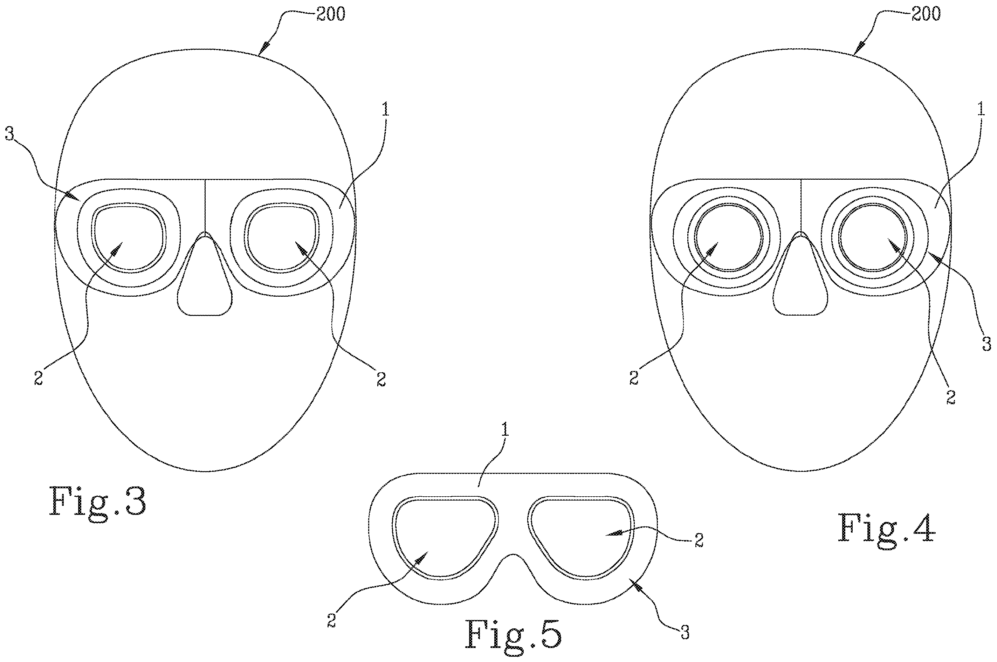

Advantageously, as illustrated in Figures 3-5, the recesses 2 and the housings 8 may have different geometrical shapes, allowing the insertion of lenses 9 having different geometries and preferably shaped to match them.

The second portion 5 may be operatively connected to the connecting portion 7 to define a locking of the above-mentioned lens 9.

According to a possible embodiment and as illustrated in Figure 10, the second portion 5 is made as one piece with the connecting portion 7; the portions 5 and 7 form a housing 8 designed to house the lens 9.

For example, the lens 9 is locked inside the housing 8 by a lobe 10 of the connecting portion 7.

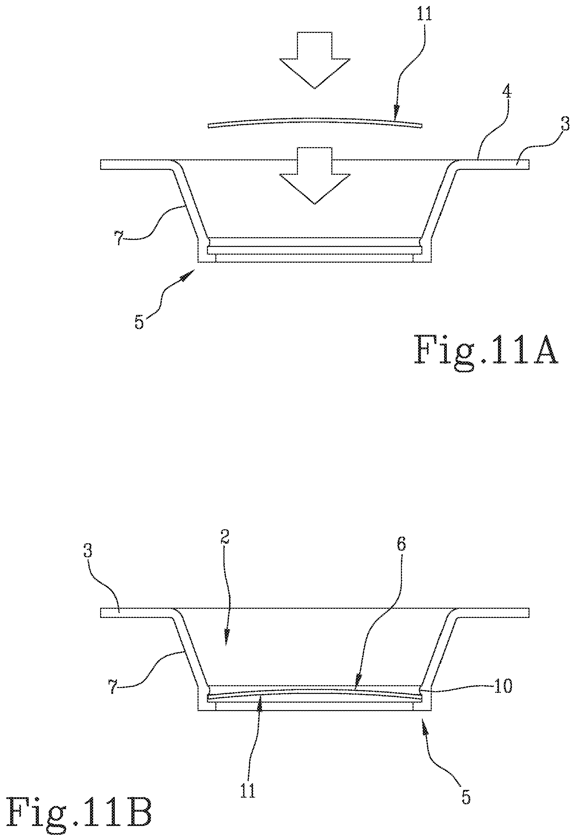

According to a possible embodiment and as illustrated in Figures 11A, 11B, the visor 1 and/or the second portion 5 comprises a removable part 11 forming the second protective surface 6.

Advantageously, the removable part 11 may be a prescription lens designed to increase the view of a user 200.

In one embodiment, the lenses 9 themselves define the protective surfaces 6.

The portion 5 may be, for example, annular in shape and operatively connected to the connecting portion 7 for fixing the respective lens 9, for example by a snap-on fitting or a threaded coupling.

The visor 1 may comprise fixing means 12 applicable to each connecting portion 7, inside the respective recess 2, for fixing the lens to the connecting portion 7 and/or to said second portion 5.

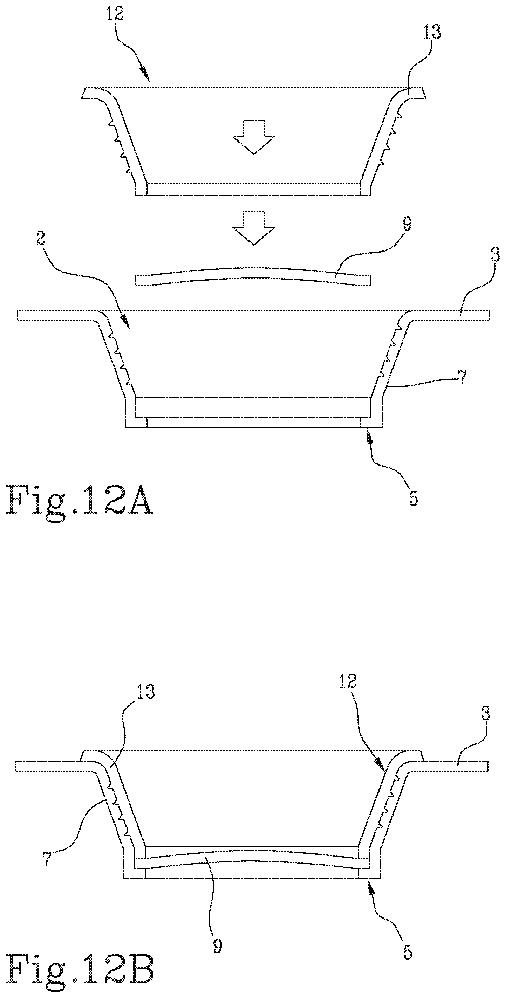

As illustrated in Figures 12A, 12B, the fixing means 12 may comprise an insert 13, in particular cylindrical or truncated cone, shaped to match the connecting portion 7 and stably anchored to it.

More specifically, the insert 13 has outer protrusions or recesses configured to be stably anchored, respectively, with outer recesses or protrusions of the connecting portion.

According to some possible embodiments of this invention, each of the second portions 5 and/or the connecting portions 7 comprises a receiving seat 14 for applying a respective lens 9 positioned behind the second portion 5, that is to say, facing the user 200. For example, the lens 9 may consist of the removable portion 11 of the second portion 5 forming the above-mentioned second protective surface 6.

In other words, the receiving seat 14 is designed to receive a lens along a direction which extends away from the user 200.

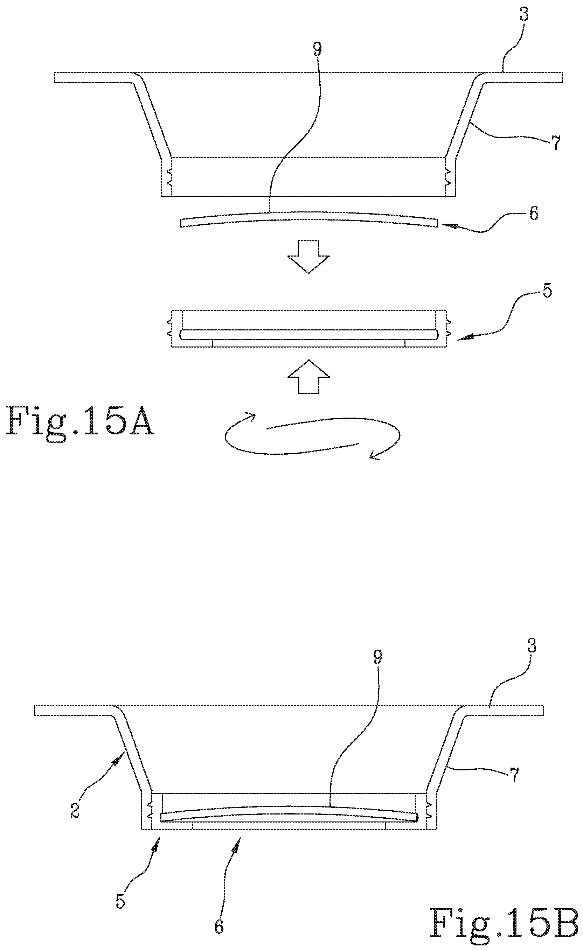

According to the embodiments, for example illustrated in Figures 13A- 15B, the second portions 5 are connectable to the connecting portions 7 by a shape coupling, for example of the threaded type (Figures 13A, 13B, 15A, 15B) or by slotting into a lobed profile (Figures 14A, 14B).

According to these embodiments, the lens 9 is inserted in the receiving seat 14 along a direction which, in use, extends away from the user 200.

Moreover, the visor 1 may comprise sealing means (not illustrated in the accompanying drawings) acting between the lens 9 and the connecting portion 7 and/or the second portion 5 configured to prevent a flow of fluid towards the eyes of the user 200.

Preferably, the sealing means comprise a hydraulic seal, for example an elastomeric O-ring.

According to another aspect, the invention relates to a safety device 100 for the face of a user 200 comprising a frame 101 configured to fix the safety device 100 to the user 200 and a visor 1 connected to the frame for protecting at least a portion of the face of the user 200.

Preferably, the safety device comprises a pair of prescription lenses 9, each inserted or insertable in a respective recess 2 and/or in a respective seat 14 of the visor 1.

In one embodiment, the visor comprises a single lens located in a single space defined by the protective surfaces 6 joined to each other.

It should be noted, therefore, that the invention achieves the preset aims by providing a protective visor, in particular for a safety device for the face of a user, and a safety device for the face of a user which is able to impart high levels of comfort to a user thanks to the presence of a pair of recesses for positioning, in a position close to the eyes of a user, of viewing and/or viewing amplification instruments.

The recesses of the visor allow the user to use any enlargement optics, both telescopic and microscopic, guaranteeing a correct focussing distance.

The visor allows compliance with the standards and dimensions of the traditional protective devices.

This feature also simplifies the work and increases the operating efficiency of a user.

Advantageously, the invention has a high level of operating flexibility and may have different types of shapes in such a way as to adapt to the specific requirements of the use.

## Claims

1. A protective visor, in particular for a safety device (100) for the face of a user (200), having a pair of recesses (2), the protective visor comprising
- at least a first portion (3) forming at least a respective first protective surface (4) designed to protect the periocular zones of the user (200);
- a pair of second portions (5) of substantially transparent material which delimit in the protective visor, at least partly, said recesses (2), and
- connecting portions (7) between said first portion (3) and said second portion (5), configured to define a surface continuity of the protective visor forming a protection for the face of the user (200), the second portions (5) and the connecting portions (7) forming the recesses (2), each second portion (5) being stably applicable to the respective connecting portion (7), the protective visor being **characterized in that** said recesses (2) are adapted to protrude from the first portion (3) towards the eyes of the user (200), for the positioning, in a position close to the eyes of the user (200), of viewing and/or viewing amplification instruments.

2. The protective visor according to claim 1, comprising:
- a pair of second protective surfaces (6) designed to protect the ocular zones of the user;
said connecting portions (7) extending between said first portion (3) and said second protective surfaces (6), and
said second protective surfaces (6) and said connecting portions (7) forming at least partly said recesses (2).

3. The protective visor according to claim 2, wherein said second portions (5) form said second protective surfaces.

4. The protective visor according to any one of the preceeding claims, wherein said recesses (2) have a depth of between 2 and 50 mm, preferably 20 mm relative to said first portion (3).

5. The protective visor according to any one of the preceeding claims, wherein said connecting portions (7) have a tubular shape or an extension converging from said first protective surface (4) to said second protective surfaces (6).

6. The protective visor according to any one of claims 2 to 5, wherein said connecting portions (7) are made as one piece with the second protective surfaces (6).

7. The protective visor according to any one of preceeding claims, wherein each connecting portion (7) is stably applicable to the first portion (3) by means of a snap-on connection, by pressure, by screwing or by gluing.

8. The protective visor according to any one of claims 1 to 6, wherein said connecting portions (7) are made as one piece with the first portion (3) using a transparent material.

9. The protective visor according to any one of preceeding claims, wherein each of said connecting portions (7) forms a housing (8) designed to receive at least one lens (9), preferably a prescription lens (9).

10. The protective visor according to any one of claims 2 to 9, comprising a removable part (11) forming said second protective surface (6), preferably said removable part (11) being a prescription lens (9).

11. The protective visor according to claim 9, wherein said second portion (5) and said connecting portion (7) are operatively connected to form a locking of said removable part (11).

12. The protective visor according to any one of preceeding claims, comprising fixing means (12) applicable to each connecting portion (7), inside the respective recess (2), for constraining a removable part (11) in said recess (2).

13. The protective visor according to claim 12, wherein said fixing means comprise an insert (13), in particular cylindrical or truncated cone, shaped to match the connecting portion (7) and stably anchored to it.

14. The protective visor according to claim 13, wherein said insert has outer protrusions or recesses designed to be stably anchored, respectively, with outer recesses or protrusions of the connecting portion.

15. The protective visor according to any one of preceeding claims, wherein each of said recesses (2) comprises a receiving seat (14) for applying a respective lens (9) positioned behind said second protective surfaces (6), that is to say, facing the side of the user (200).

16. The protective visor according to claim 10, comprising sealing means, preferably a hydraulic seal, at least active between said removable part (11) and said connecting portion (7) for preventing a flow of fluid towards the eyes of the user (200).

17. The protective visor according to any one of claims 2 to 16, wherein said second protective surfaces (6) each comprise a respective lens (9).

18. The protective visor according to any one of claims 2 to 17, wherein said second portions (5) define said second protective surfaces (6),
said second portions (5) and said connecting portions (7) forming said recesses (2).

19. A safety device for the face of a user (200) comprising:
- a frame (101) configured to fix said safety device to a user;
- a protective visor (1) according to any one of the preceding claims, connected to said frame (101) for protecting at least a portion of the face of the user (200).

20. The safety device according to claim 19, wherein said protective visor (1) comprises at least one prescription lens (9) in one of said recesses (2).

21. The protective visor according to claim 2, wherein the protective surfaces (6) are joined together, forming a single space.

## Patentansprüche

1. Schutzvisier, insbesondere für eine Sicherheitsvorrichtung (100) für das Gesicht eines Benutzers (200), aufweisend ein Paar Ausnehmungen (2), wobei das Schutzvisier Folgendes umfasst
- mindestens einen ersten Abschnitt (3), der mindestens eine jeweilige erste Schutzoberfläche (4) bildet, die ausgestaltet ist, um die Augenbereichszonen des Benutzers (200) zu schützen;
- ein Paar zweiter Abschnitte (5) aus im Wesentlichem transparentem Material, die im Schutzvisier zumindest teilweise die Ausnehmungen (2) begrenzen, und
- Verbindungsabschnitte (7) zwischen dem ersten Abschnitt (3) und dem zweiten Abschnitt (5), die ausgelegt sind, um eine Oberflächenkontinuität des Schutzvisiers zu definieren, bildend einen Schutz für das Gesicht des Benutzers (200), wobei die zweiten Abschnitte (5) und die Verbindungsabschnitte (7) die Ausnehmungen (2) bilden und ein jeder zweite Abschnitt (5) stabil am jeweiligen Verbindungsabschnitt (7) anbringbar ist, wobei das Schutzvisier **dadurch gekennzeichnet ist, dass** die Ausnehmungen (2) eingerichtet sind, um aus dem ersten Abschnitt (3) hinführend zu den Augen des Benutzers (200) für das Positionieren von Betrachtungs- und/oder Betrachtungsverstärkungsinstrumenten in einer Position in der Nähe der Augen des Benutzers (200) hervorzuspringen.

2. Schutzvisier nach Anspruch 1, umfassend:
- ein Paar zweiter Schutzoberflächen (6), die ausgestaltet sind, um die Augenzonen des Benutzers zu schützen,
wobei sich die Verbindungsabschnitte (7) zwischen dem ersten Abschnitt (3) und den zweiten Schutzoberflächen (6) erstrecken und
die zweiten Schutzoberflächen (6) und die Verbindungsabschnitte (7) zumindest teilweise die Ausnehmungen (2) bilden.

3. Schutzvisier nach Anspruch 2, wobei die zweiten Abschnitte (5) die zweiten Schutzoberflächen bilden.

4. Schutzvisier nach einem der vorhergehenden Ansprüche, wobei die Ausnehmungen (2) eine Tiefe zwischen 2 und 50 mm, vorzugsweise 20 mm, relativ zum ersten Abschnitt (3) aufweisen.

5. Schutzvisier nach einem der vorhergehenden Ansprüche, wobei die Verbindungsabschnitte (7) eine rohrförmige Form oder eine Ausdehnung aufweisen, die von der ersten Schutzoberfläche (4) zu den zweiten Schutzoberflächen (6) zusammenläuft.

6. Schutzvisier nach einem der Ansprüche 2 bis 5, wobei die Verbindungsabschnitte (7) einstückig mit den zweiten Schutzoberflächen (6) ausgeführt sind.

7. Schutzvisier nach einem der vorhergehenden Ansprüche, wobei ein jeder Verbindungsabschnitt (7) stabil am ersten Abschnitt (3) mittels einer Einschnappverbindung, durch Druck, durch Schrauben oder durch Verkleben anbringbar ist.

8. Schutzvisier nach einem der Ansprüche 1 bis 6, wobei die Verbindungsabschnitte (7) einstückig mit dem ersten Abschnitt (3) unter Verwendung von transparentem Material ausgeführt sind.

9. Schutzvisier nach einem der vorhergehenden Ansprüche, wobei ein jeder der Verbindungsabschnitte (7) eine Aufnahme (8) bildet, die ausgestaltet ist, um mindestens ein Brillenglas (9), vorzugsweise ein Korrekturglas (9) aufzunehmen.

10. Schutzvisier nach einem der Ansprüche 2 bis 9, umfassend einen entfernbaren Teil (11), der die zweite Schutzoberfläche (6) bildet, wobei der entfernbare Teil (11) vorzugsweise ein Korrekturglas (9) ist.

11. Schutzvisier nach Anspruch 9, wobei der zweite Abschnitt (5) und der Verbindungsabschnitt (7) betriebswirksam verbunden sind, um eine Verriegelung des entfernbaren Teils (11) zu bilden.

12. Schutzvisier nach einem der vorhergehenden Ansprüche, umfassend Fixiermittel (12), die an einem jeden Verbindungsabschnitt (7) innerhalb der jeweiligen Ausnehmung (2) anbringbar sind, um einen entfernbaren Teil (11) in der Ausnehmung (2) zu befestigen.

13. Schutzvisier nach Anspruch 12, wobei die Fixiermittel einen Einsatz (13), insbesondere zylindrisch oder stumpfkegelförmig, umfassen, der passend zum Verbindungsabschnitt (7) ausgeformt und stabil daran verankert ist.

14. Schutzvisier nach Anspruch 13, wobei der Einsatz äußere Vorsprünge oder Ausnehmungen aufweist, die ausgestaltet sind, um jeweils stabil mit äußeren Ausnehmungen bzw. Vorsprüngen des Verbindungsabschnitts verankert zu werden.

15. Schutzvisier nach einem der vorhergehenden Ansprüche, wobei eine jede der Ausnehmungen (2) einen Aufnahmesitz (14) zum Anbringen eines jeweiligen Brillenglases (9), das hinter den zweiten Schutzoberflächen (6), d. h. der Seite des Benutzers (200) zugewandt, positioniert ist, umfasst.

16. Schutzvisier nach Anspruch 10, umfassend Dichtungsmittel, vorzugsweise eine hydraulische Dichtung, die mindestens zwischen dem entfernbaren Teil (11) und dem Verbindungsabschnitt (7) aktiv sind, um ein Strömen von Fluid hinführend zu den Augen des Benutzers (200) zu vermeiden.

17. Schutzvisier nach einem der Ansprüche 2 bis 16, wobei die zweiten Schutzoberflächen (6) jeweils ein jeweiliges Brillenglas (9) umfassen.

18. Schutzvisier nach einem der Ansprüche 2 bis 17, wobei die zweiten Abschnitte (5) die zweiten Schutzoberflächen (6) definieren und die zweiten Abschnitte (5) und die Verbindungsabschnitte (7) die Ausnehmungen (2) bilden.

19. Sicherheitsvorrichtung für das Gesicht eines Benutzers (200), umfassend:
- einen Rahmen (101), der ausgelegt ist, um die Sicherheitsvorrichtung an einem Benutzer zu fixieren;
- ein Schutzvisier (1) nach einem der vorhergehenden Ansprüche, das mit dem Rahmen (101) verbunden ist, um mindestens einen Abschnitt des Gesichts des Benutzers (200) zu schützen.

20. Sicherheitsvorrichtung nach Anspruch 19, wobei das Schutzvisier (1) mindestens ein Korrekturglas (9) in einer der Ausnehmungen (2) umfasst.

21. Schutzvisier nach Anspruch 2, wobei die Schutzoberflächen (6) zusammengefügt sind und einen einzelnen Raum bilden.

## Revendications

1. Visière de protection, en particulier pour un dispositif de sécurité (100) pour le visage d'un utilisateur (200), comportant une paire d'évidements (2), la visière de protection comprenant
- au moins une première portion (3) formant au moins une première surface de protection respective (4) conçue pour protéger les zones périoculaires de l'utilisateur (200) ;
- une paire de secondes portions (5) de matériau substantiellement transparent qui délimitent dans la visière de protection, au moins partiellement, lesdits évidements (2), et
- des portions de connexion (7) entre ladite première portion (3) et ladite seconde portion (5), configurées pour définir une continuité de surface de la visière de protection formant une protection pour le visage de l'utilisateur (200), les secondes portions (5) et les portions de connexion (7) formant les évidements (2), chaque seconde portion (5) étant applicable de manière stable à la portion de connexion respective (7), la visière de protection étant **caractérisée en ce que** lesdits évidements (2) sont adaptés pour faire saillie de la première portion (3) vers les yeux de l'utilisateur (200), pour le positionnement, dans une position proche des yeux de l'utilisateur (200), d'instruments de visualisation et/ou d'amplification de visualisation.

2. Visière de protection selon la revendication 1, comprenant :
- une paire de secondes surfaces de protection (6) conçues pour protéger les zones oculaires de l'utilisateur ;
lesdites portions de connexion (7) s'étendant entre ladite première portion (3) et lesdites secondes surfaces de protection (6), et
lesdites secondes surfaces de protection (6) et lesdites portions de connexion (7) formant au moins partiellement lesdits évidements (2).

3. Visière de protection selon la revendication 2, dans laquelle lesdites secondes portions (5) forment lesdites secondes surfaces de protection.

4. Visière de protection selon l'une quelconque des revendications précédentes, dans laquelle lesdits évidements (2) ont une profondeur comprise entre 2 et 50 mm, de préférence 20 mm par rapport à ladite première portion (3).

5. Visière de protection selon l'une quelconque des revendications précédentes, dans laquelle lesdites portions de connexion (7) ont une forme tubulaire ou une extension convergeant de ladite première surface de protection (4) vers lesdites secondes surfaces de protection (6).

6. Visière de protection selon l'une quelconque des revendications 2 à 5, dans laquelle lesdites portions de connexion (7) sont réalisées d'une seule pièce avec les secondes surfaces de protection (6).

7. Visière de protection selon l'une quelconque des revendications précédentes, dans laquelle chaque portion de connexion (7) est applicable de manière stable à la première portion (3) au moyen d'une connexion par encliquetage, par pression, par vissage ou par collage.

8. Visière de protection selon l'une quelconque des revendications 1 à 6, dans laquelle lesdites portions de connexion (7) sont réalisées d'une seule pièce avec la première portion (3) en utilisant un matériau transparent.

9. Visière de protection selon l'une quelconque des revendications précédentes, dans laquelle chacune desdites portions de connexion (7) forme un logement (8) conçu pour recevoir au moins une lentille (9), de préférence une lentille de prescription (9).

10. Visière de protection selon l'une quelconque des revendications 2 à 9, comprenant une partie amovible (11) formant ladite seconde surface de protection (6), de préférence ladite partie amovible (11) étant une lentille de prescription (9).

11. Visière de protection selon la revendication 9, dans laquelle ladite seconde portion (5) et ladite portion de connexion (7) sont fonctionnellement reliées pour former un verrouillage de ladite partie amovible (11).

12. Visière de protection selon l'une quelconque des revendications précédentes, comprenant des moyens de fixation (12) applicables à chaque portion de connexion (7), à l'intérieur de l'évidement respectif (2), pour contraindre une partie amovible (11) dans ledit évidement (2).

13. Visière de protection selon la revendication 12, dans laquelle lesdits moyens de fixation comprennent un insert (13), en particulier cylindrique ou tronconique, formé pour correspondre à la portion de connexion (7) et ancré de manière stable à celle-ci.

14. Visière de protection selon la revendication 13, dans laquelle ledit insert comporte des saillies ou des évidements extérieurs conçus pour être ancrés de manière stable, respectivement, avec des évidements ou des saillies extérieurs de la portion de connexion.

15. Visière de protection selon l'une quelconque des revendications précédentes, dans laquelle chacun desdits évidements (2) comprend un siège de réception (14) pour appliquer une lentille respective (9) positionnée derrière lesdites secondes surfaces de protection (6), c'est-à-dire faisant face au côté de l'utilisateur (200) .

16. Visière de protection selon la revendication 10, comprenant des moyens d'étanchéité, de préférence un joint hydraulique, au moins actif entre ladite partie amovible (11) et ladite portion de connexion (7) pour empêcher un écoulement de liquide vers les yeux de l'utilisateur (200).

17. Visière de protection selon l'une quelconque des revendications 2 à 16, dans laquelle lesdites secondes surfaces de protection (6) comprennent chacune une lentille respective (9).

18. Visière de protection selon l'une quelconque des revendications 2 à 17, dans laquelle lesdites secondes portions (5) définissent lesdites secondes surfaces de protection (6), lesdites secondes portions (5) et lesdites portions de connexion (7) formant lesdits évidements (2).

19. Dispositif de sécurité pour le visage d'un utilisateur (200), comprenant :
- un cadre (101) configuré pour fixer ledit dispositif de sécurité à un utilisateur ;
- une visière de protection (1) selon l'une quelconque des revendications précédentes, reliée audit cadre (101) pour protéger au moins une portion du visage de l'utilisateur (200).

20. Dispositif de sécurité selon la revendication 19, dans lequel ladite visière de protection (1) comprend au moins une lentille de prescription (9) dans l'un desdits évidements (2).

21. Visière de protection selon la revendication 2, dans laquelle les surfaces de protection (6) sont jointes ensemble, formant un seul espace.
